# EUROPÄISCHE PATENTSCHRIFT

(11) **EP 2 990 026 B1**
(45) Veröffentlichungstag und Bekanntmachung des Hinweises auf die Patenterteilung: **05.04.2017**
(21) Anmeldenummer: 14182366.6
(22) Anmeldetag: 27.08.2014
(51) Int. Cl.: A61K 8/44, A61K 8/46, C11D 1/37, C11D 11/00, A61Q 5/02, A61Q 19/10, C11D 1/10, C11D 1/28, C11D 3/00

(54) **Wässrige Tensid-Zusammensetzungen**
Aqueous tenside compositions
Compositions de tensioactifs aqueuses

(43) Veröffentlichungstag der Anmeldung: 02.03.2016
(73) Patentinhaber: BASF SE, 67056 Ludwigshafen am Rhein (DE)
(72) Erfinder: Behler, Ansgar, 46240 Bottrop (DE); Brunn, Claudia, 40597 Düsseldorf (DE); Stanislowski, Detlev, 40822 Mettmann (DE)
(74) Vertreter: Reinhardt, Jürgen

(56) Entgegenhaltungen:
- EP-A1- 2 468 842
- DE-A1- 4 009 096

## Beschreibung

### Gebiet der Erfindung

Die vorliegende Erfindung betrifft wässrige Tensid-Zusammensetzungen mit einem Gehalt an alpha-Sulfofettsäuredisalzen sowie speziellen N-Acyl-Glutaminsäure-Verbindungen.

### Stand der Technik

Anionische Tenside gehören zu den am weitesten verbreiteten grenzflächenaktiven Verbindungen und werden außer in Wasch- und Reinigungsmittel auch auf dem Gebiet der Kosmetik vielfältig eingesetzt. Übliche anionische Tenside, wie sie vor allem in der Kosmetik eingesetzt werden, sind die Salze von Alkylethersulfaten (Alkylpolyethersulfate, Fettalkoholpolyglycolethersulfate, verkürzt auch Ethersulfate). Sie zeichnen sich durch starkes Schaumvermögen, hohe Reinigungskraft, geringe Härte- und Fettempfindlichkeit aus und finden vielfach Verwendung zur Herstellung von kosmetischen Produkten wie beispielsweise Haarshampoos, Schaumoder Duschbädern, aber auch in Handgeschirrspülmitteln.

Für viele aktuelle Anwendungen werden an anionische Tenside außer einer guten grenzflächenaktiven Wirkung weitere Anforderungen gestellt. Insbesondere in der Kosmetik ist eine hohe dermatologische Verträglichkeit erforderlich. Des Weiteren ist in der Regel eine ausreichende Wasserlöslichkeit, eine gute Verträglichkeit mit möglichst vielen der in der Kosmetik eingesetzten Wirk- und Hilfsstoffen, ein gutes Schaumvermögen und eine gute Verdickbarkeit erwünscht. Des Weiteren besteht ein Bedarf an anionischen Tensiden, die zumindest teilweise aus biogenen Quellen und speziell auch nachwachsenden Rohstoffen hergestellt werden können. Weiterhin besteht auch ein Bedarf an Tensiden, die keine alkoxilierten Gruppen aufweisen und die somit insbesondere den Einsatz von Ethylenoxid zu ihrer Herstellung überflüssig machen.

Die sogenannten alpha-Sulfofettsäuredisalze ("Disalze") sind eine bekannte Tensidklasse, die jedoch eine sehr schlechte Wasserlöslichkeit aufweisen (vergl. z.B. F.Schambil und M.J.Schwuger, Tenside Surf. Det. 27 (1990), 6 S. 380-385): So beträgt etwa die Wasserlöslichkeit von C14-Di-Na-Salz bei 20°C lediglich 0,7% (vergl. die Graphik auf S. 381). Dies ist für die Praxis, etwa kosmetische Zubereitungen unbefriedigend gering. Dementsprechend haben Disalze bislang in wässrigen tensidischen Formulierungen keine Anwendung als anionisches Basistensid gefunden und wurden auf Grund ihrer schlechten Wasserlöslichkeit nicht für die Herstellung transparenter, stabiler Formulierungen eingesetzt.

E.G. Su et al. (SÖFW Journal 139 4-2013, Seiten 30 und 32 -36) beschreiben Formulierungen mit sulfatfreien hautmilden Reinigungs-Zusammensetzungen enthaltend Aminosäure-basierte Tenside. Dabei werden drei Typen Aminosäure-basierter Tenside untersucht, nämlich Glycinate, Sarkosinate und Glutamate. Die Untersuchung kommt zu dem Schluss, dass diese drei Typen von Tensiden sich als Substitute für Laurylethersulfate in Reinigungsmitteln eignen. Im Fokus des Artikels sind die Glycinate, während die Glutamate nur pauschal erwähnt werden. Über eine mögliche Kombination von Glutamaten mit alpha-Sulfofettsäure-Disalzen ist nichts offenbart.

Untersuchungen der Anmelderin haben gezeigt, dass das Schaumvermögen von Aminosäurebasierten Tensiden, insbesondere von N-Acyl-Glutamaten stark pH-abhängig ist und über weite pH-Bereiche für kosmetische Rinse-Off-Formulierungen unzureichend ist. Unter Rinse-Off-Formulierungen sind Formulierungen für Duschbäder, Haarshampoos und dergleichen zu verstehen, bei denen es auf gutes Schaumverhalten, insbesondere exzellentes Anschäumverhalten ankommt und wo nach Verwendung ein Abspülen von der Haut erfolgt.

Untersuchungen der Anmelderin haben ferner gezeigt, dass Aminosäure-basierte Tenside, insbesondere N-Acyl-Glutamate sich bei niedrigen pH-Werten nur schwer in stabile, transparente Formulierungen einarbeiten lassen.

### Beschreibung der Erfindung

Die komplexe Aufgabe der vorliegenden Erfindung hat darin bestanden, wässrige Tensid-Zusammensetzungen bereitzustellen, die sich durch die im Folgenden genannten Eigenschaften auszeichnen, wobei jede dieser Eigenschaften ein technisches Merkmal darstellt:
- Gute Transparenz, worunter im Rahmen der vorliegenden Erfindung zu verstehen ist, dass die wässrigen Tensid-Zusammensetzungen bei der quantitativen Bestimmung mittels eines TurbiScan MA 2000 (Messgerät der Firma Formulaction) bei 23°C eine mittlere Transmission von wenigstens 80%, vorzugsweise von wenigstens 85% und insbesondere von wenigstens 88% aufweisen.
- Gutes Schaumvermögen. Hierzu sei angemerkt, dass im Bereich der Kosmetik unter Schaumvermögen verschiedene Aspekte verstanden werden können, wobei insbesondere Schaumvolumen, Schaumstabilität, Schaumelastizität, Wassergehalt des Schaumes, optische Merkmale des Schaumes wie beispielsweise die Porengröße, sowie die Schaum-Sensorik zur Beurteilung des Schaumes herangezogen werden können. Besonders wünschenswert ist, dass eine tensidische Formulierung ein hohes Schaumvolumen während des Anschäumens aufweist. Das Anschäumverhalten spielt insbesondere bei sogenannten Rinse-off-Produkten, worunter Produkte zu verstehen sind, die beim Reinigen oder Pflegen mit der Haut in Berührung kommen, dann aber wieder abgewaschen werden (z.B. Duschgele, Duschformulierungen, Shampoos, Flüssigseifen, usw.) eine sehr wichtige Rolle. Ganz besonders in diesem Bereich ist ein möglichst hohes Schaumvolumens erwünscht. In der Praxis findet das Anschäumen in einem relativ kurzen Zeitraum statt (von wenigen Sekunden bis zu einer Minute). Typischerweise wird beim Anschäumen ein Duschgel oder ein Shampoo durch Reiben zwischen Händen, Haut und/oder Haaren verteilt und zum Schäumen gebracht. Ein exzellentes Anschäumverhalten ist im Rahmen der vorliegenden Erfindung von grundlegender Bedeutung. Im Labor kann das Anschäumverhalten einer wässrigen Tensidlösung z.B. dadurch beurteilt werden, dass man in einer vergleichbar kurzen Zeitspanne die Lösung durch Rühren, Schütteln, Pumpen, Durchperlen eines Gasstroms oder auf andere Weise in Bewegung versetzt. Der im Rahmen der vorliegenden Erfindung verwendete Schaumtest ist im Beispielteil näher beschrieben.
- Hydrolysestabilität bei pH-Werten von 8 oder weniger.
- Lagerstabilität bei Raumtemperatur (23 °C) über mindestens 12 Wochen, ohne dass irgendwelche sichtbaren Veränderungen (etwa Austrübungen, Verfärbungen, Phasentrennungen, Verlust der Transparenz und dergleichen) auftreten.

Gegenstand der Erfindung sind zunächst wässrige Tensid-Zusammensetzungen enthaltend
- ein oder mehrere **alpha-Sulfofettsäuredisalze (A)** der allgemeinen Formel (I),

   R¹CH(SO₃M¹)COOM² (I),

   worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamine,
- ein oder mehrere **N-Acyl-Glutaminsäure-Verbindungen (B)** der allgemeinen Formel (II),

   M³OOC-CH₂-CH₂-CH(NH-CO-R²)-COOM⁴ (II)

   worin der Rest R² einen linearen oder verzweigten Alkyl- oder Alkenylrest mit 7 bis 19 C-Atomen bedeutet und die Reste M³ und M⁴ - unabhängig voneinander - ausgewählt werden aus der Gruppe H, Li, Na, K, Ca/2, Mg/2, Ammonium und Alkanolamine
- **Wasser,**
wobei folgende Maßgaben gelten:
- der Gehalt der wässrigen Tensid-Zusammensetzungen an den Verbindungen (A) und (B) liegt - bezogen auf die gesamte wässrige Tensid-Zusammensetzung - bei mindestens 5 Gew.-%;
- sofern die wässrigen Tensid-Zusammensetzungen ein oder mehrere Estersulfonate (E) der allgemeinen Formel (V),

   R⁵CH(SO₃M⁷)COOR⁶ (V)

   worin der Rest R⁵ einen linearen oder verzweigten Alkyl- oder Alkenylrest mit 6 bis 18 C-Atomen bedeutet und der Rest R⁶ einen linearen oder verzweigten Alkyl- oder Alkenylrest mit 1 bis 20 C-Atomen bedeutet, wobei der Rest R⁶ logischerweise erst ab 3 C-Atomen ein Alkenylrest sein oder verzweigt sein kann, und der Rest M⁷ ausgewählt wird aus der Gruppe Li, Na, K, Ca/2, Mg/2, Ammonium und Alkanolamine, enthält, gilt, dass die Verbindungen (A) - bezogen auf die Gesamtheit der Verbindungen (A) und (E) - zu 50 Gew.-% oder mehr - und insbesondere zu 90 Gew.-% oder mehr - vorliegen müssen;
- das Gewichtsverhältnis der Verbindungen (B) : (A) in den wässrigen Tensid-Zusammensetzungen liegt im Bereich von 2 : 1 bis 6 : 1;
- der pH-Wert der wässrigen Tensid-Zusammensetzungen liegt bei 8 oder weniger;
- die mittlere Transmission der wässrigen Tensid-Zusammensetzungen bei 23°C - gemessen mit einem TurbiScan MA 2000 - liegt bei wenigstens 80%.

Überraschenderweise wurde die obengenannte komplexe Aufgabe durch die erfindungsgemäßen Tensid-Zusammensetzungen in ausgezeichneter Weise gelöst. Es war nicht vorhersehbar und mithin höchst überraschend, dass sich Disalze (A) bei Kombination mit den N-Acyl-Glutaminsäure-Verbindungen (B) in erheblich höheren Konzentrationen einsetzen lassen, erkennbar daran, dass die wässrigen Zusammensetzungen transparent sind und nicht trübe. Mit anderen Worten: Die Löslichkeit der Disalze (A) erfährt durch die Kombination mit den N-Acyl-Glutaminsäure-Verbindungen (B) eine signifikante Steigerung. Die erfindungsgemäßen-Zusammensetzungen zeigen auch ein synergistisches Schaumverhalten: Während beide Tenside einzeln ein für den Rinse-Off-Anwendungsbereich wenig befriedigendes Schaumverhalten aufweisen, zeigen die erfindungsgemäßen Kombinationen der Tenside (A) und (B) ein exzellentes Anschäumverhalten.

### Zu den Verbindungen (A)

Die Verbindungen (A), die im Rahmen der vorliegenden Erfindung als **alpha-Sulfofettsäuredisalze** bezeichnet werden, sind für die erfindungsgemäßen wässrigen Tensid-Zusammensetzungen obligatorisch. Sie haben die oben angegebene Formel (I)

R¹CHCSO₃M¹)COOM² (I),

worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin.

In einer Ausführungsform gilt die Maßgabe, dass der Anteil der Verbindungen (A) in den wässrigen Tensid-Zusammensetzungen, bei denen der Rest R¹ ein Alkenylrest ist, - bezogen auf die Gesamtmenge der Verbindungen (A) - bei 3 Gew.-% oder weniger liegt

In einer bevorzugten Ausführungsform bedeutet der Rest R¹ in der Formel (I) einen gesättigten, linearen Rest mit 10 bis 16 C-Atomen, wobei in Bezug auf die Verbindungen (A) gilt, dass der Anteil der Verbindungen (A), bei denen der Rest R¹ ein Decyl- und/oder ein Dodecylrest ist , - bezogen auf die Gesamtmenge der Verbindungen (A) - bei 90 Gew.-% oder mehr liegt.

Vorzugsweise bedeuten die Reste M¹ und M² in der Formel (I) Na.

Die Verbindungen (A) können nach allen dem Fachmann einschlägig bekannten Methoden hergestellt werden. Eine besonders bevorzugte Methode der Herstellung ist dabei die Sulfierung der entsprechenden Carbonsäuren. Dabei setzt man die entsprechenden Carbonsäure und insbesondere die entsprechenden Fettsäuren mit gasförmigem Schwefeltrioxid um, wobei man das Schwefeltrioxid vorzugsweise in einer Menge einsetzt, dass das molare Verhältnis von SO₃ zu Fettsäure im Bereich von 1,0 : 1 bis 1,1 : 1 liegt. Die so erhaltenen Rohprodukte, die saure Sulfierprodukte darstellen, werden anschließend partiell oder vollständig neutralisiert, wobei eine vollständige Neutralisation mit wässriger NaOH bevorzugt ist. Gewünschtenfalls können auch Reinigungsschritte und/oder eine Bleiche (zur Einstellung der gewünschten hellen Farbe der Produkte) vorgenommen werden.

In einer besonders bevorzugten Ausführungsform werden die Verbindungen (A) in technischer Form eingesetzt. Dies bedeutet, dass man die entsprechenden Carbonsäuren, insbesondere native Fettsäure, mit gasförmigem Schwefeltrioxid sulfiert, wodurch nach partieller oder vollständiger Neutralisation der entstehenden sauren Sulfierprodukte ein Gemisch der Verbindungen (A), (C) und (D) resultiert. Durch entsprechende Einstellungen der Reaktionsparameter (insbesondere Mol-Verhältnis von Carbonsäure und Schwefeltrioxid sowie Reaktionstemperatur) lässt sich das Verhältnis der Verbindungen (A), (C) und (D) steuern. Die Verbindungen (C) und (D) sind unten im Kapitel "Bevorzugte Ausführungsformen" beschrieben.

Im Rahmen der vorliegenden Erfindung sind solche technischen Mischungen der alpha-Sulfofettsäuredisalze bevorzugt, die wie folgt zusammengesetzt sind:
- Der Gehalt an (A) liegt im Bereich von 60 bis 100 Gew.%,
- der Gehalt an (C) liegt im Bereich von 0 bis 20 Gew.%,
- der Gehalt an (D) liegt im Bereich von 0 bis 20 Gew.%,
mit der Maßgabe, dass die Summe der Komponenten (A), (C) und (D) in dieser Mischung 100 Gew.% beträgt.

Ganz besonders sind solche technischen Mischungen bevorzugt, bei wie folgt zusammengesetzt sind:
- Der Gehalt an (A) liegt im Bereich von 70 bis 80 Gew.%,
- der Gehalt an (C) liegt im Bereich von 10 bis 15 Gew.%,
- der Gehalt an (D) liegt im Bereich von 10 bis 15 Gew.%,
mit der Maßgabe, dass die Summe der Komponenten (A), (C) und (D) in dieser Mischung 100 Gew.% beträgt.

### Zu den Verbindungen (B)

Die Verbindungen (B), die im Rahmen der vorliegenden Erfindung als **N-Acyl-Glutaminsäure-Verbindungen** bezeichnet werden, sind für die erfindungsgemäßen wässrigen Tensid-Zusammensetzungen obligatorisch. Sie haben die oben angegebene Formel (II)

M³OOC-CH₂-CH₂-CH(NH-CO-R²)-COOM⁴ (II)

worin der Rest R² einen linearen oder verzweigten Alkyl- oder Alkenylrest mit 7 bis 19 C-Atomen bedeutet und die Reste M³ und M⁴ - unabhängig voneinander - ausgewählt werden aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin. In einer Ausführungsform bedeuten die Reste M³ und M⁴ jeweils Na (Natrium).

In einer Ausführungsform gilt die Maßgabe, dass der Anteil der Verbindungen (B), bei denen der Rest R² ein Alkenylrest ist, - bezogen auf die Gesamtmenge der Verbindungen (B) in den wässrigen Tensid-Zusammensetzungen - bei 3 Gew.-% oder weniger liegt.

Die Verbindungen (B) können nach allen dem Fachmann einschlägig bekannten Methoden hergestellt werden.

In einer Ausführungsform bedeutet Rest R² in der Formel (II) einen gesättigten, linearen Rest mit 11 bis 13 C-Atomen.

### Bevorzugte Ausführungsformen

In einer Ausführungsform enthalten die erfindungsgemäßen wässrigen Tensid-Zusammensetzungen neben den Verbindungen (A), (B) und Wasser zusätzlich ein oder mehrere **Verbindungen (C)** der allgemeinen Formel (III)

R⁴COOM⁵ (III)

In der Formel (III) bedeutet der Rest R⁴ einen linearen oder verzweigten Alkyl- oder Alkenylrest mit 7 bis 19 C-Atomen und der Reste M⁵ wird ausgewählt aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin.

In einer Ausführungsform enthalten die erfindungsgemäßen wässrigen Tensid-Zusammensetzungen neben den Verbindungen (A), (B) und Wasser zusätzlich ein oder mehrere **anorganische Salze der Schwefelsäure (D)** der allgemeinen Formel (IV)

(M⁶)₂SO₄ (IV)

wobei M⁶ ausgewählt wird aus der Gruppe Li, Na, K, Ca/2, Mg/2, Ammonium und Alkanolamin. Besonders bevorzugte Alkanolamine sind dabei Monoethanolamin, Diethanolamin, Triethanolamin und Mono-Isopropanolamin.

In einer bevorzugten Ausführungsform enthalten die erfindungsgemäßen wässrigen Tensid-Zusammensetzungen die Verbindungen (A), (B), (C) und (D). Dabei ist es besonders bevorzugt, wenn die Reste M¹ und M² der Verbindungen (A), die Reste M³ und M⁴ der Verbindungen (B), der Rest M⁵ der Verbindungen (C) und der Rest M⁶ der Verbindungen (D) die Bedeutung Na (Natrium) haben.

Wie oben ausgeführt liegt der Gehalt der Zusammensetzungen an den Verbindungen (A) und (B) - bezogen auf die gesamte Zusammensetzung - bei mindestens 5 Gew.-%. Vorzugsweise liegt der Gehalt der Zusammensetzungen an den Verbindungen (A) und (B) - bezogen auf die gesamte Zusammensetzung - im Bereich von 5 bis 50 Gew.-% , insbesondere im Bereich von 5 bis 20 Gew.-% und besonders bevorzugt im Bereich von 8 bis 12 Gew.-%.

Wie oben ausgeführt liegt das Gewichtsverhältnis der Verbindungen (B) : (A) in den Zusammensetzungen im Bereich von 2 : 1 bis 6 : 1.
In einer bevorzugten Ausführungsform liegt das Gewichtsverhältnis der Verbindungen (B) : (A) in den Zusammensetzungen im Bereich von 3 : 1 bis 4,5 : 1.

Der pH-Wert der Zusammensetzungen liegt bei 8 oder weniger. Vorzugsweise wird er auf einen Wert von 7 oder weniger eingestellt. In einer bevorzugten Ausführungsform liegt der pH-Wert der Zusammensetzungen im Bereich von 4,5 bis 7,0 und insbesondere 4,5 bis 5,5.

Gewünschtenfalls können die erfindungsgemäßen wässrigen Tensid-Zusammensetzungen zusätzlich ein oder mehrere weitere Tenside enthalten, die strukturell nicht zu den oben genannten Verbindungehn (A), (B), (D) oder (E) zählen. Bei diesen Tensiden kann es sich um anionische, kationische, nichtionische oder amphotere Tenside handeln.

### Verwendung der Zusammensetzungen

Ein weiterer Erfindungsgegenstand ist die Verwendung der oben genannten Zusammensetzungen für kosmetische Mittel, sowie Wasch- und Reinigungsmittel.

Im Hinblick auf kosmetische Mittel sind dabei insbesondere solche besonders bevorzugt, die in Form von Haarshampoos, Duschgelen, Seifen, Syndets, Waschpasten, Waschlotionen, Scrub-Präparate, Schaumbädern, Ölbädern, Duschbädern, Rasierschäumen, Rasierlotionen, Rasiercremes und Zahnpflegeprodukten (etwa Zahnpasten, Mundwässern und dergleichen) vorliegen.

Im Hinblick auf Reinigungsmittel sind dabei insbesondere Mittel mit niedrigem pH-Wert zur Reinigung harter Oberflächen bevorzugt, wie Bad- und WC-Reiniger und dergleichen, sowie für Reinigungs- und/oder Duftgele zur Anwendung in sanitären Einrichtungen.

### Beispiele

### Eingesetzte Substanzen

### VE-Wasser = vollentsalztes Wasser

**SFA-I:** alpha-Sulfofettsäuredisalz technischer Qualität auf Basis von nativer C_{12/14}-Fettsäure; Zusammensetzung: 74 Gew.% Dinatrium-2-Sulfolaurat, 13 Gew.% Natrium-Laurat, 11 Gew.% Natriumsulfat, 2 Gew.-% Wasser. Die Bezeichnung "Laurat" bedeutet hierbei, dass das C_{12/14}-Gewichtsverhältnis der Mischung der zu Grunde liegenden nativen Fettsäuren 70 : 30 beträgt.

**SFA-II:** aufgereinigtes alpha-Sulfofettsäuredisalz auf Basis von nativer C_{12/14}-Fettsäure; Zusammensetzung: 90 Gew.% Dinatrium-2-Sulfolaurat, 5 Gew.% Natrium-Laurat, 0,2 Gew.% Natriumsulfat, 4,8 Gew.-% Wasser. Die Bezeichnung "Laurat" bedeutet hierbei, dass das C_{12/14}-Gewichtsverhältnis der Mischung der zu Grunde liegenden nativen Fettsäuren 70 : 30 beträgt.

**ACG:** Plantapon ACG HC (Sodium Cocoyl Glutamat, 41 Gew.-% Aktivsubstanz), Handelsprodukt der Fa. BASF PCN).

### Mess- und Prüfmethoden

**pH-Wert:** Unter Einsatz eines handelsüblichen pH-Meters, wurde der pH-Wert direkt in der Formulierung, also der wässrigen Tensid-Zusammensetzung, gemessen.

**Homogenität und Aussehen:** Die Beurteilung der Homogenität und des Aussehens der wässrigen Tensid-Zusammensetzungen erfolgte visuell (mit bloßem Auge) in 125ml Weithals-Glasflaschen. Beurteilt wurde dabei zunächst die Homogenität. Unter Homogenität wird im Rahmen der vorliegenden Erfindung verstanden, dass keine mit bloßem Auge sichtbaren Aufrahmung oder ein Bodensatz auftritt. Sofern die Zusammensetzungen als homogen beurteilt wurden, wurde auch ihr Aussehen beurteilt und beispielsweise mit Attributen wie leicht opak (jedoch immer noch deutlich durchscheinend) bis wasserklar charakterisiert.

### Transparenz:

Die Proben wurden zunächst mit bloßem Auge betrachtet. Offensichtliche Trübungen, Kristalle, Bodensätze werden dabei direkt erkannt. War die Probe augenscheinlich frei von Feststoffausfällungen, erfolgte eine quantitative Bestimmung der Transparenz mittels eines TurbiScan MA 2000 (Messgerät der Firma Formulaction) bei 23°C. Die Proben wurden als transparent bezeichnet, wenn sie eine mittlere Transmission von wenigstens 80% aufwiesen. Die Angabe "transparent" in der unten stehenden Tabelle 1 bedeutet dementsprechend, dass die mittlere Transmission wenigstens 80% betrug.

Zur quantitativen Bestimmung der Transparenz mit einem TurbiScan MA 2000 wurden dabei zunächst 5ml-Proben der zu prüfenden wässrigen Tensid-Zusammensetzungen in die gerätespezifische Messzelle gegeben und für 24 Stunden bei Raumtemperatur (23°C) stehen gelassen bis alle Luftblasen ausgetreten waren. Danach wurde die Transmission des eingestrahlten Lichts (Wellenlänge 850 nm) über eine Probenhöhe von 20 mm bis 50 mm gemessen. Die Auswertung erfolgte mit der vom Hersteller des Messgeräts mitgelieferten Software Turbisoft (Version 1.2.1.): Für jede Messung wird von der Software ein Mittelwert der Transmission (in %) über die Probenhöhe ausgegeben. Dieser Mittelwert wird im Rahmen der vorliegenden Anmeldung mittlere Transmission genannt. Dabei wurde die Transmissionsmessung bei jeder Probe 3-mal wiederholt und aus den dabei erhaltenen Werten für die mittlere Transmission der zahlenmäßige Mittelwert gebildet.

### Bestimmung des Anschäumverhaltens:

Zur Prüfung des Anschäumverhaltens (Rotorschaum-Methode) wurde ein handelsübliches Messgerät eingesetzt (Sita Foam Tester R-2000). Dabei wurde zunächst eine wässrige Tensidlösung wie folgt hergestellt: 1 g Aktivsubstanz der jeweils zu prüfenden Probe (als Proben wurden SFA-I oder ACG oder Mischungen dieser Substanzen eingesetzt, siehe unten; beim SFA-I wird - wie oben angegeben - unter Aktivsubstanzgehalt der Disalzgehalt verstanden) wurde bei 20 °C in 1 Liter vollentsalztem Wasser (= Wasser mit einem Härtegrad von 0 °dH) gelöst. Der pH-Wert der Lösung wurde mit verdünnter Citronensäure bzw. Natronlauge auf den gewünschten Wert - der jeweilige Wert ist den Tabellen 1 und 2 zu entnehmen - eingestellt. Die so hergestellt Lösung wurde temperiert auf 30°C.

Aus der temperierten Vorlage wurden 250ml in das Messgerät überführt und bei einer Umdrehungszahl von 1300 Umdrehungen pro Minute wurde für 10 Sekunden aufgeschäumt, das dann vorliegende Schaumvolumen (in ml) ermittelt, dann weitere 10 Sekunden geschäumt, das dann vorliegende Schaumvolumen (in ml) ermittelt, usw., d.h. nach jeweils 10 Sekunden währendem Aufschäumen wurde die Schaumhöhe bestimmt. Nach 80 Sekunden Aufschäumzeit wurde die Messung beendet. Die Messung wurde bei jeder Probe 3-mal mit jeweils frischer Lösung aus dem gleichen Ansatz wiederholt und das Ergebnis der Messungen nach 40, 60 und 80 Sekunden als Mittelwert aus diesen drei Messungen angegeben (siehe Tabelle).

### Lagerstabilität:

Die Tensid-Zusammensetzungen wurden über einen Zeitraum von 12 Wochen bei 23°C gelagert. Danach erfolgte die Prüfung der beiden Parameter Homogenität und Aussehen der Zusammensetzungen. Die Zusammensetzungen wurden dann als lagerstabil angesehen, wenn beide Parameter über den gesamten Zeitraum von 12 Wochen unverändert blieben.

### Beispiele

### Beispiel 1 (erfindungsgemäß):

Herstellung (Ansatzgröße 200g): Die Komponenten gemäß Tabelle 1 wurden unter Rühren bei 23 °C in VE-Wasser (= vollentsalztes Wasser = Wasser mit einem Härtegrad von 0 °dH) gelöst. Danach erfolgte die Einstellung des pH-Werts durch Zugabe von Citronensäure (50%ige Lösung) auf den in Tabelle 1 angegebenen Wert. Die beurteilten Parameter (Homogenität, Aussehen, Lagerstabilität) können der Tabelle 1 entnommen werden.

### Beispiel 2 (erfindungsgemäß):

Herstellung wie Beispiel 1, jedoch mit geänderten Mengen der eingesetzten Komponenten (siehe Tabelle 1). pH-Wert Einstellung wie in Beispiel 1 mit Citronensäure (auf den in Tabelle 1 angegebenen Wert). Die beurteilten Parameter (Homogenität, Aussehen, Lagerstabilität) können der Tabelle 1 entnommen werden.

**Tabelle 1**

| | Beispiel 1 | Beispiel 2 |
|---|---|---|
| ACG | 43,9 g | 47,9 g |
| SFA I | 8,1 g | 5,9 g |
| VE-Wasser | 148,0 g | 146,2 g |
| pH Wert | 4,6 | 4,6 |
| Homogenität | homogen | homogen |
| Aussehen | transparent | transparent |
| Lagerstabilität | stabil | stabil |
| Aktivsubstanzgehalt | 12% | 12% |
| Gewichtsverhältnis Aktivsubstanz | 3:1 | 4,5:1 |
| ACG : SFA I | | |

### Vergleichsbeispiele 1 und 2

Herstellung wie Beispiel 1, jedoch wurde ausschließlich ACG bzw. SFA I eingesetzt (siehe Tabelle 2). pH-Wert Einstellung erfolgte mit Citronensäure (auf den in Tabelle 2 angegebenen Wert). Die beurteilten Parameter (Homogenität, Aussehen, Lagerstabilität) können der Tabelle 2 entnommen werden.

**Tabelle 2**

| | Vergleichsbeispiel 1 | Vergleichsbeispiel 2 |
|---|---|---|
| ACG | 43,9 g | |
| SFA I | | 32,4 g |
| VE-Wasser | 156,1 g | 167,6 g |
| pH Wert | 4,6 | 4,5 |
| Homogenität | inhomogen | inhomogen |
| Aussehen | weißer, kristalliner Bodensatz | weißer Bodensatz |
| Aktivsubstanzgehalt | 9% | 6% |
| Gewichtsverhältnis Aktivsubstanz | 1:0 | 0:1 |
| ACG : SFA I | | |

### Beispiele 3 bis 7 (erfindungsgemäß):

Die Daten zum Anschäumverhalten von Tensid-Mischungen ACG/SFA I bzw. ACG/SFA II können Tabelle 3 entnommen werden.

**Tabelle 3**

| | Beispiel 3 | Beispiel 4 | Beispiel 5 | Beispiel 6 | Beispiel 7 |
|---|---|---|---|---|---|
| ACG | 1,83 g | 1,95 g | 1,95 g | 1,99 g | 1,99 g |
| SFA I | 0,34 g | 0,27 g | 0,27 g | 0,24 g | |
| SFA II | | | | | 0,20 g |
| Gewichtsverhältnis Aktivsubstanz | 3:1 | 4:1 | 4:1 | 4,5:1 | 4,5:1 |
| ACG : SFA | | | | | |
| VE-Wasser | 1000 ml | 1000ml | 1000ml | 1000ml | 1000ml |
| pH Wert | 5,5 | 4,0 | 5,0 | 6,9 | 5,5 |
| Schaumvolumen nach 40 sek | 426 ml | 597 ml | 480 ml | 391 ml | 477 ml |
| Schaumvolumen nach 60 sek | 672 ml | 835 ml | 808 ml | 543 ml | 794 ml |
| Schaumvolumen nach 80 sek | 854 ml | 851 ml | 869 ml | 746 ml | 857 ml |

### Vergleichsbeispiele 3 bis 7:

Die Daten zum Anschäumverhalten der Einzeltenside ACG bzw. SFA I können der Tabelle 4 entnommen werden.

**Tabelle 4**

| | Vergleichsbeispiel 3 | Vergleichsbeispiel 4 | Vergleichsbeispiel 5 | Vergleichsbeispiel 6 | Vergleichsbeispiel 7 |
|---|---|---|---|---|---|
| ACG | 2,44 g | 2,44 g | 2,44 g | | |
| SFA I | | | | 1,35 g | 1,35 g |
| Gewichtsverhältnis Aktivsubstanz | 1:0 | 1:0 | 1:0 | 0:1 | 0:1 |
| ACG : SFA | | | | | |
| VE-Wasser | 1000 ml | 1000ml | 1000ml | 1000ml | 1000ml |
| pH Wert | 4,0 | 5,5 | 7,0 | 5,5 | 7,0 |
| Schaumvolumen nach 40 sek | 205 ml | 201 ml | 110 ml | 148 ml | 271 ml |
| Schaumvolumen nach 60 sek | 261 ml | 285 ml | 166 ml | 188 ml | 369 ml |
| Schaumvolumen nach 80 sek | 304 ml | 365 ml | 232 ml | 215 ml | 460 ml |

## Patentansprüche

1. Wässrige Tensid-Zusammensetzungen enthaltend
• ein oder mehrere **alpha-Sulfofettsäuredisalze** (**A**) der allgemeinen Formel (I),
R¹CHCSO₃M¹)COOM² (I)
worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamin,
• ein oder mehrere **N-Acyl-Glutaminsäure-Verbindungen** (B) der allgemeinen Formel (II),
M³OOC-CH₂-CH₂-CH(NH-CO-R²)-COOM⁴ (II)
worin der Rest R² einen linearen oder verzweigten Alkyl- oder Alkenylrest mit 7 bis 19 C-Atomen bedeutet und die Reste M³ und M⁴ - unabhängig voneinander - ausgewählt werden aus der Gruppe H, Li, Na, K, Ca/2, Mg/2, Ammonium und Alkanolamine,
• **Wasser,**
wobei folgende Maßgaben gelten:
• der Gehalt der wässrigen Tensid-Zusammensetzungen an den Verbindungen (A) und (B) liegt - bezogen auf die gesamte wässrigen Tensid-Zusammensetzung - bei mindestens 5 Gew.-%;
• sofern die wässrigen Tensid-Zusammensetzungen ein oder mehrere Estersulfonate (E) der allgemeinen Formel (V),
R⁵CH(SO₃M⁷)COOR⁶ (V)
worin der Rest R⁵ einen linearen oder verzweigten Alkyl- oder Alkenylrest mit 6 bis 18 C-Atomen bedeutet und der Rest R⁶ einen linearen oder verzweigten Alkyl- oder Alkenylrest mit 1 bis 20 C-Atomen bedeutet, wobei der Rest R⁷ logischerweise erst ab 3 C-Atomen ein Alkenylrest sein oder verzweigt sein kann, und der Rest M⁷ ausgewählt wird aus der Gruppe Li, Na, K, Ca/2, Mg/2, Ammonium und Alkanolamine, enthält, gilt, dass die Verbindungen (A) - bezogen auf die Gesamtheit der Verbindungen (A) und (E) - zu 50 Gew.-% oder mehr vorliegen müssen;
• das Gewichtsverhältnis der Verbindungen (B) : (A) in den wässrigen Tensid-Zusammensetzungen liegt im Bereich von 2 : 1 bis 6 : 1;
• der pH-Wert der wässrigen Tensid-Zusammensetzungen liegt bei 8 oder weniger;
• die mittlere Transmission der wässrigen Tensid-Zusammensetzungen bei 23°C - gemessen mit einem TurbiScan MA 2000 - liegt bei wenigstens 80%.

2. Zusammensetzungen nach Anspruch 1, wobei der Rest R² in der Formel (II) ein gesättigter Alkylrest mit 11 bis 13 C-Atomen ist.

3. Zusammensetzungen nach Anspruch 1 oder 2, wobei der Rest R¹ in der Formel (I) einen gesättigten, linearen Rest mit 10 bis 16 C-Atomen bedeutet, wobei in Bezug auf die Verbindungen (A) gilt, dass der Anteil der Verbindungen (A), bei denen der Rest R¹ ein Decyl- oder ein Dodecylrest ist , - bezogen auf die Gesamtmenge der Verbindungen (A) - bei 90 Gew.-% oder mehr liegt.

4. Zusammensetzungen nach einem der Ansprüche 1 bis 3, wobei die Reste M¹ und M² Na bedeuten.

5. Zusammensetzungen nach einem der Ansprüche 1 bis 4, wobei die Reste M³ und M⁴ Na bedeuten

6. Zusammensetzungen nach einem der Ansprüche 1 bis 5, wobei die Zusammensetzungen zusätzlich ein oder mehrere **Verbindungen (C)** der allgemeinen Formel (III)
R⁴COOM⁵ (III)
enthält, worin der Rest R⁴ einen linearen oder verzweigten Alkyl- oder Alkenylrest mit 7 bis 19 C-Atomen bedeutet und der Reste M⁵ ausgewählt wird aus der Gruppe H, Li, Na, K, Ca/2, Mg/2, Ammonium und Alkanolamin.

7. Zusammensetzungen nach einem der Ansprüche 1 bis 6, wobei die Zusammensetzungen zusätzlich ein oder mehrere **anorganische Salze der Schwefelsäure (D)** der allgemeinen Formel (IV)
(M⁶)₂SO₄ (IV)
Enthält, wobei M⁶ ausgewählt wird aus der Gruppe Li, Na, K, Ca/2, Mg/2, Ammonium und Alkanolamin.

8. Zusammensetzungen nach einem der Ansprüche 1 bis 7, wobei der Gehalt der Zusammensetzungen an den Verbindungen (A) und (B) - bezogen auf die gesamte Zusammensetzung - im Bereich von 8 bis 12 Gew.-% liegt.

9. Zusammensetzungen nach einem der Ansprüche 1 bis 8, wobei das Gewichtsverhältnis der Verbindungen (B) : (A) in den Zusammensetzungen im Bereich von 3 : 1 bis 4,5 : 1 liegt.

10. Zusammensetzungen nach einem der Ansprüche 1 bis 9, wobei der pH-Wert der Zusammensetzungen im Bereich von 4,0 bis 7,0 liegt.

11. Zusammensetzungen nach einem der Ansprüche 1 bis 9, wobei der pH-Wert der Zusammensetzungen im Bereich von 4,5 bis 5,5 liegt

12. Verwendung der Zusammensetzungen nach einem der Ansprüche 1 bis 11 für kosmetische Mittel sowie Wasch- und Reinigungsmittel.

13. Verwendung der Zusammensetzungen nach einem der Ansprüche 1 bis 11 für kosmetische Mittel in Form von Haarshampoos, Duschgelen, Seifen, Syndets, Waschpasten, Waschlotionen, Scrub-Präparate, Schaumbädern, Ölbädern, Duschbädern, Rasierschäumen, Rasierlotionen, Rasiercremes und Zahnpflegeprodukten.

14. Verwendung der Zusammensetzungen nach einem der Ansprüche 1 bis 11 für Mittel mit niedrigem pH-Wert zur Reinigung harter Oberflächen, wie Bad- und WC-Reiniger und dergleichen, sowie für Reinigungs- und/oder Duftgele zur Anwendung in sanitären Einrichtungen.

## Claims

1. An aqueous surfactant composition comprising
• one or more **alpha-sulfo fatty acid disalts (A)** of the general formula (I),
R¹CH (SO₃M¹) COOM² (I)
in which the radical R¹ is a linear or branched alkyl or alkenyl radical with 6 to 18 carbon atoms and the radicals M¹ and M² - independently of one another - are selected from the group H, Li, Na, K, Ca/2, Mg/2, ammonium and alkanolamine,
• one or more **N-acylglutamic acid compounds (B)** of the general formula (II),
M³OOC-CH₂-CH₂-CH (NH-CO-R²) -COOM⁴ (II)
in which the radical R² is a linear or branched alkyl or alkenyl radical with 7 to 19 carbon atoms and the radicals M³ and M⁴ - independently of one another - are selected from the group H, Li, Na, K, Ca/2, Mg/2, ammonium and alkanolamines,
• **water,**
where the following provisos apply:
• the content of the compounds (A) and (B) in the aqueous surfactant composition is - based on the total aqueous surfactant composition - at least 5% by weight;
• if the aqueous surfactant composition comprises one or more **ester sulfonates (E)** of the general formula (V),
R⁵CH (SO₃M⁷) COOR⁶ (V)
in which the radical R⁵ is a linear or branched alkyl or alkenyl radical with 6 to 18 carbon atoms and the radical R⁶ is a linear or branched alkyl or alkenyl radical with 1 to 20 carbon atoms, where the radical R⁷ can logically be an alkenyl radical or be branched only above 3 carbon atoms, and the radical M⁷ is selected from the group Li, Na, K, Ca/2, Mg/2, ammonium and alkanolamines, it is the case that the compounds (A) - based on the totality of the compounds (A) and (E) - must be present to 50% by weight or more;
• the weight ratio of the compounds (B) : (A) in the aqueous surfactant composition is in the range from 2 : 1 to 6 : 1;
• the pH of the aqueous surfactant composition is 8 or less;
• the average transmission of the aqueous surfactant composition at 23°C - measured using a TurbiScan MA 2000 - is at least 80%.

2. The composition according to claim 1, wherein the radical R² in the formula (II) is a saturated alkyl radical with 11 to 13 carbon atoms.

3. The composition according to claim 1 or 2, wherein the radical R¹ in the formula (I) is a saturated, linear radical with 10 to 16 carbon atoms, where with regard to the compounds (A) it is the case that the fraction of the compounds (A) in which the radical R¹ is a decyl or a dodecyl radical - based on the total amount of the compounds (A) - is 90% by weight or more.

4. The composition according to any one of claims 1 to 3, wherein the radicals M¹ and M² are Na.

5. The composition according to any one of claims 1 to 4, wherein the radicals M³ and M⁴ are Na.

6. The composition according to any one of claims 1 to 5, wherein the composition comprises additionally one or more **compounds (C)** of the general formula (III)
R⁴COOM⁵ (III)
in which the radical R⁴ is a linear or branched alkyl or alkenyl radical with 7 to 19 carbon atoms and the radicals M⁵ is selected from the group H, Li, Na, K, Ca/2, Mg/2, ammonium and alkanolamine.

7. The composition according to any one of claims 1 to 6, wherein the composition comprises additionally one or more **inorganic salts of sulfuric acid (D)** of the general formula (IV)
(M⁶)₂SO₄ (IV)
wherein M⁶ is selected from the group Li, Na, K, Ca/2, Mg/2, ammonium and alkanolamine.

8. The composition according to any one of claims 1 to 7, wherein the content of the compounds (A) and (B) in the composition - based on the total composition - is in the range from 8 to 12% by weight.

9. The composition according to any one of claims 1 to 8, wherein the weight ratio of the compounds (B) : (A) in the composition is in the range from 3 : 1 to 4.5 : 1.

10. The composition according to any one of claims 1 to 9, wherein the pH of the composition is in the range from 4.0 to 7.0.

11. The composition according to any one of claims 1 to 9, wherein the pH of the composition is in the range from 4.5 to 5.5.

12. The use of the composition according to any one of claims 1 to 11 for cosmetic products and also detergents and cleaners.

13. The use of the composition according to any one of claims 1 to 11 for cosmetic products in the form of hair shampoos, shower gels, soaps, syndets, washing pastes, washing lotions, scrub preparations, foam baths, oil baths, shower baths, shaving foams, shaving lotions, shaving creams and dental care products.

14. The use of the composition according to any one of claims 1 to 11 for products with a low pH for cleaning hard surfaces, such as bath and toilet cleaners and the like, and also for cleaning and/or fragrance gels for use in sanitary installations.

## Revendications

1. Compositions aqueuses de tensioactifs, contenant :
- un ou plusieurs disels d'acides alpha-sulfo-gras (A) de formule générale (I)
R¹CH (SO₃M¹) COOM² (I)
dans laquelle le radical R¹ signifie un radical alkyle ou alcényle linéaire ou ramifié de 6 à 18 atomes C et les radicaux M¹ et M² sont choisis, indépendamment l'un de l'autre, dans le groupe constitué par H, Li, Na, K, Ca/2, Mg/2, ammonium et alcanolamine,
- un ou plusieurs composés d'acide N-acyl-glutamique (B) de formule générale (II)
M³OOC-CH₂-CH₂-CH(NH-CO-R²)-COOM⁴ (II)
dans laquelle le radical R² signifie un radical alkyle ou alcényle linéaire ou ramifié de 7 à 19 atomes C et les radicaux M³ et M⁴ sont choisis, indépendamment l'un de l'autre, dans le groupe constitué par H, Li, Na, K, Ca/2, Mg/2, ammonium et alcanolamine,
- de l'eau,
les conditions suivantes étant respectées :
- la teneur des compositions aqueuses de tensioactifs en composés (A) et (B), par rapport à la composition aqueuse de tensioactifs totale, est d'au moins 5 % en poids ;
- si les compositions aqueuses de tensioactifs contiennent un ou plusieurs ester-sulfonates (E) de formule générale (V)
R⁵CH (SO₃M⁷) COOR⁶ (V)
dans laquelle le radical R⁵ signifie un radical alkyle ou alcényle linéaire ou ramifié de 6 à 18 atomes C et le radical R⁶ signifie un radical alkyle ou alcényle linéaire ou ramifié de 1 à 20 atomes C, le radical R⁷ ne pouvant logiquement être un radical alcényle ou être ramifié qu'à partir de 3 atomes C, et le radical M⁷ est choisi dans le groupe constitué par Li, Na, K, Ca/2, Mg/2, ammonium et alcanolamine, les composés (A) doivent être présents, par rapport à la totalité des composés (A) et (E), à hauteur de 50 % en poids ou plus ;
- le rapport en poids des composés (B):(A) dans les compositions aqueuses de tensioactifs se situe dans la plage allant de 2:1 à 6:1 ;
- le pH des compositions aqueuses de tensioactifs est de 8 ou moins ;
- la transmission moyenne des compositions aqueuses de tensioactifs à 23 °C, mesurée avec un TurbiScan MA 2000, est d'au moins 80 %.

2. Compositions selon la revendication 1, dans lesquelles le radical R² dans la formule (II) est un radical alkyle saturé de 11 à 13 atomes C.

3. Compositions selon la revendication 1 ou 2, dans lesquelles le radical R¹ dans la formule (I) signifie un radical linéaire saturé de 10 à 16 atomes C, en ce qui concerne les composés (A), la proportion de composés (A) dans lesquels le radical R¹ est un radical décyle ou dodécyle, par rapport à la quantité totale des composés (A), étant de 90 % en poids ou plus.

4. Compositions selon l'une quelconque des revendications 1 à 3, dans lesquelles les radicaux M¹ et M² signifient Na.

5. Compositions selon l'une quelconque des revendications 1 à 4, dans lesquelles les radicaux M³ et M⁴ signifient Na.

6. Compositions selon l'une quelconque des revendications 1 à 5, dans lesquelles les compositions contiennent en outre un ou plusieurs composés (C) de formule générale (III)
R⁴COOM⁵ (III)
dans laquelle le radical R⁴ signifie un radical alkyle ou alcényle linéaire ou ramifié de 7 à 19 atomes C et les radicaux M⁵ est choisi dans le groupe constitué par H, Li, Na, K, Ca/2, Mg/2, ammonium et alcanolamine.

7. Compositions selon l'une quelconque des revendications 1 à 6, dans lesquelles les compositions contiennent en outre un ou plusieurs sels inorganiques de l'acide sulfurique (D) de formule générale (IV)
(M⁶)₂SO₄ (IV)
dans laquelle M⁶ est choisi dans le groupe constitué par Li, Na, K, Ca/2, Mg/2, ammonium et alcanolamine.

8. Compositions selon l'une quelconque des revendications 1 à 7, dans lesquelles la teneur des compositions en composés (A) et (B), par rapport à la composition totale, se situe dans la plage allant de 8 à 12 % en poids.

9. Compositions selon l'une quelconque des revendications 1 à 8, dans lesquelles le rapport en poids des composés (B):(A) dans les compositions se situe dans la plage allant de 3:1 à 4,5:1.

10. Compositions selon l'une quelconque des revendications 1 à 9, dans lesquelles le pH des compositions se situe dans la plage allant de 4,0 à 7,0.

11. Compositions selon l'une quelconque des revendications 1 à 9, dans lesquelles le pH des compositions se situe dans la plage allant de 4,5 à 5,5.

12. Utilisation des compositions selon l'une quelconque des revendications 1 à 11 pour des agents cosmétiques, ainsi que des détergents.

13. Utilisation des compositions selon l'une quelconque des revendications 1 à 11 pour des agents cosmétiques sous la forme de shampoings capillaires, de gels douche, de savons, de détersifs synthétiques, de pâtes nettoyantes, de lotions nettoyantes, de préparations de gommage, de bains moussants, de bains d'huile, de bains douches, de mousses de rasage, de lotions de rasage, de crèmes de rasage et de produits de soin dentaire.

14. Utilisation des compositions selon l'une quelconque des revendications 1 à 11 pour des agents à pH faible pour le nettoyage de surfaces dures, tels que des produits de nettoyage pour salles de bain et WC et analogues, ainsi que des gels nettoyants et/ou parfumés destinés à une utilisation dans des aménagements sanitaires.
